# FASCICULE DE BREVET EUROPEEN

(11) **EP 1 248 772 B1**
(45) Date de publication et mention de la délivrance du brevet: **22.03.2006**
(21) Numéro de dépôt: 01907650.4
(22) Date de dépôt: 19.01.2001
(51) Int. Cl.: C07D 251/34, C08G 18/16

(54) **PROCEDE D'OBTENTION DE POLYISOCYANATE(S) RAMIFIE(S) FAIBLEMENT COLORE(S), ET COMPOSITION EN DECOULANT**
VERFAHREN ZUR HERSTELLUNG VON SCHWACH GEFÄRBTEN, VERZWEIGTEN POLYISOCYANATEN UND DARAUS RESULTIERENDE ZUSAMMENSETZUNG
METHOD FOR OBTAINING SLIGHTLY COLOURED BRANCHED POLYISOCYANATE(S), AND RESULTING COMPOSITION

(30) Priorité: 20.01.2000 FR 0000688; 04.05.2000 FR 0005736
(43) Date de publication de la demande: 16.10.2002
(73) Titulaire: RHODIA CHIMIE, 92512 Boulogne Billancourt Cedex (FR)
(72) Inventeur: BERNARD, Jean-Marie, F-69440 Mornant (FR); DALLEMER, Frédéric, F-69003 Lyon (FR); REVELANT, Denis, F-69740 Genas (FR)
(74) Mandataire: Colombet, Alain André
(86) Numéro de dépôt international: PCT/FR2001/000186
(87) Numéro de publication internationale: WO 2001/053277

(56) Documents cités:
- EP-A- 0 010 589
- EP-A- 0 668 271
- EP-A- 0 780 418
- EP-A- 0 818 485
- WO-A-99/23128
- DE-A- 4 405 054
- US-A- 5 489 663
- US-A- 5 691 440
- CHEMICAL ABSTRACTS, vol. 131, no. 24, 13 décembre 1999 (1999-12-13) Columbus, Ohio, US; abstract no. 323875, KOJIMA, KATSUYA ET AL: "Manufacture of isocyanurate-containing polyisocyanates as curing agents" XP002150287 & JP 11 302351 A (MITSUI CHEMICALS INC., JAPAN) 2 novembre 1999 (1999-11-02)

## Description

La présente invention a pour objet un procédé de préparation de polyisocyanates-polyisocyanurates par cyclotrimérisation catalytique de polyisocyanates monomères. Elle concerne plus particulièrement la cyclotrimérisation de fonctions isocyanates portées par des carbones d'hybridation sp³ de nature particulière. Ces carbones sont des carbones, soit en position néopentylique, c'est-à-dire que ledit carbone est relié à un radical tertiaire tel que le tertiobutyle, soit que ledit carbone est un carbone secondaire ou tertiaire, de préférence entrant dans un cycle aliphatique.

La cyclotrimérisation des isocyanates est connue depuis plusieurs décennies. Ces trimérisations mettent en général en oeuvre des mécanismes de nature basique. Ces bases peuvent être de nature ionique comme les hydroxydes ou les sels de base forte et d'acide faible. Ces bases peuvent également être des bases non ioniques mais présentant un doublet très accessible et très basique.

Les premières trimérisations réalisées l'ont été sur les isocyanates aromatiques, c'est-à-dire les isocyanates portés par un carbone membre d'un noyau aromatique. Ces trimérisations ne présentent pas de difficulté particulière et l'on trouve aisément des catalyseurs susceptibles de réaliser facilement des trimérisations d'isocyanates aromatiques.

Les problèmes inhérents aux isocyanates aromatiques ont conduit à développer des isocyanates aliphatiques qui présentent des propriétés chimiques et des propriétés physiques distinctes et souvent très améliorées par rapport aux isocyanates aromatiques.

La plupart des isocyanates aliphatiques sont trop volatils pour être utilisés tels quels. Aussi est-on obligé d'alourdir leurs molécules en réalisant soit des oligomères (biurets, trimères, ...), soit des oligo-condensats avec des polyols.

La trimérisation des isocyanates aliphatiques à chaîne linéaire est désormais bien maîtrisée et l'on possède des catalyseurs capables de réaliser des trimérisations dans de bonnes conditions, c'est-à-dire avec de bons rendements et en développant peu de coloration.

En revanche, pour des raisons qui ne sont pas complètement élucidées, la trimérisation d'isocyanates aliphatiques ramifiés et, notamment, d'isocyanates cycloaliphatiques ou en position néopentylique, reste difficile d'une part, en raison de la difficulté à trouver des catalyseurs donnant des rendements et une productivité satisfaisants et, d'autre part, parce que ces dérivés ont une forte propension à développer en leur sein des colorations indésirables.

Les problèmes de coloration, ainsi que cela a été mentionné précédemment, ont déjà été traités pour les isocyanates aliphatiques linéaires tels que l'HDI (hexaméthylènediisocyanate) mais les techniques utilisées sont difficilement transposables aux isocyanates de nature ramifiée tels que les isocyanates en positions néopentylique, cycloaliphatique et tertiaire, et ce, en particulier, parce que la réactivité de ces isocyanates ramifiés est très significativement moindre de celle des isocyanates linéaires, en sorte que les catalyseurs utilisés pour les isocyanates aliphatiques linéaires donnent souvent des rendements faibles, voire très faibles, dans le cas des isocyanates ramifiés.

Ainsi, on a proposé d'utiliser comme anion basique un anion fluorure pour réaliser des cyclotrimérisations d'aliphatiques linéaires sans coloration mais cette technique est difficilement utilisable pour les cycloaliphatiques car les rendements sont très significativement moindres dans leur cas.

Le dégazage de monomères isocyanates a par ailleurs déjà été décrit en vue de la cyclotrimérisation d'HDI.

Ainsi, EP 330 966 décrit un procédé de trimérisation d'HDI à l'aide d'un hydroxyde d'ammonium quaternaire dans lequel l'HDI de départ est débarrassé du gaz carbonique jusqu'à une teneur résiduelle inférieure à 20 ppm en poids, ceci afin de réduire la quantité de catalyseur à une valeur inférieure à 0,03 % en poids dans le but de diminuer la coloration du milieu réactionnel final.

EP 524 501 décrit un procédé de préparation de polyisocyanates comprenant des groupes isocyanurates et allophanates à partir d'HDI en mettant en oeuvre en tant que catalyseur un hydroxyde de triméthylbenzytammonium ou un hydroxyde d'ammonium quaternaire dans lequel les substituants sont des groupes alkyles en C₁-C₂₀ substitués éventuellement par des groupes hydroxyles. Il est précisé dans ce document que le mélange d'HDI de départ comprend moins de 10 ppm de CO₂.

Les catalyseurs illustrés dans ce document comportent toutefois tous trois substituants méthyle, le quatrième étant soit un groupe benzyle, soit un groupe hydroxyalkyle.

US 5,232,988 décrit un procédé de préparation de polyisocyanates masqués comprenant la réaction de trimérisation de diisocyanates cydiques tel que l'IPDI en présence de carboxylates, phénolates ou hydroxydes d'ammoniums quaternaires dans lequel le diisocyanate de départ est traité par bullage d'un gaz inerte.

US 5,914,383 décrit la préparation d'une composition polyisocyanate comprenant des trimères de type iminooxadazine dione et éventuellement des groupes isocyanurates en présence d'un polyfluorure en tant que catalyseur.

Ce document décrit en particulier la préparation d'une composition du type précité à partir d'HDI et d'IPDI dans laquelle, avant la réaction catalytique à proprement parler, on élimine les gaz dissous dans le mélange d'isocyanates de départ.

DE 197 54 748 et EP 927 731 décrivent des procédés de préparation de polyisocyanates à partir d'IPDI mettant en oeuvre des monomères de départ ayant de faibles teneurs en chlore, obtenus notamment par le procédé dit "urée".

A l'inverse, EP 379 914 et US 5,013,838 préconisent l'addition de dioxyde de carbone lors de la trimérisation de diisocyanates organiques aliphatiques et/ou cycloaliphatiques, mettant en oeuvre un catalyseur consistant en un fluorure d'ammonium ou de phosphonium.

JP 11 302,351 divulgue un procédé de préparation de polyisocyanates comprenant des isocyanurates, ne présentant pas de décoloration après exposition aux UV, en utilisant des composés hydroxylés en présence d'hydroxydes d'ammonium comme catalyseurs.

Dans EP 0 818 485 A2 est décrit un procédé d'obtention de polyisocyanates comprenant des isocyanurates, en utilisant des catalyseurs de type chlorures d'ammonium, et dans US 5,691,440, des catalyseurs de type carboxylàtes d'ammonium. Les contre-ions de ces catalyseurs correspondent à des acides forts, tout comme dans EP 0 668 271 A1.

La publication de demande de brevet DE 44 05 054 divulgue quant à elle des compositions polyisocyanates comprenant des fonctions uréthane, allophanate, urée, biuret, carbodi-imide, urétidione et/ou isocyanurate, ces dernières étant obtenues par cyclotrimérisation au moyen de catalyseurs comportant des fonctions hydroxyle.

EP 0 780 418 A2 divulgue des préparations pour la polymérisation de polyamines, à base de polyisocyanates aromatiques trimérisés.

C'est pourquoi un des buts de la présente invention est de fournir un procédé qui permette l'obtention de trimères d'isocyanates ramifiés en évitant de développer une coloration indésirable.

Un autre but de la présente invention est de fournir un procédé qui permette de bons rendements et une bonne productivité.

Un autre but de la présente invention est de fournir un procédé qui permette la trimérisation d'isocyanates cycloaliphatiques et notamment de l'IPDI (souvent désigné par l'appellation d'isophoronediisocyanate).

Un autre but de la présente invention est de fournir un procédé qui permette la trimérisation d'isocyanates cycloaliphatiques en améliorant la réactivité du système catalytique utilisé à cet effet.

Ces buts, et d'autres qui apparaîtront par la suite, sont atteints au moyen d'un procédé de trimérisation de monomères isocyanates aliphatiques ramifiées par action d'un catalyseur à base d'ammonium ou de phosphonium quaternaire dont la somme des carbones est au plus égale à 30 et au moins égale à 12 ; dont le contre-ion est choisi parmi les anions correspondant à des acides faibles dont le p*K*_{A} est au moins égal à 8, de préférence à 10, plus préférentiellement à 12 ; dont les substituants sont aliphatiques (c'est à dire sont reliés à l'atome porteur de la charge positive par un atome de carbone d'hybridation sp³) et ne possèdent pas d'insaturation en bêta (comme en ont les positions benzylique, allylique ou propargylique); et que lesdits oniums sont introduits sous forme de mélange aqueux et comportent au plus 2 radicaux de plus de 6 atomes, avantageusement de plus de 10 atomes, de préférence de plus de 12 atomes de carbone, et qu'aucun substituant dudit onium n'est porteur de fonction hydroxyle.

L'invention a également pour objet un procédé de préparation de polyisocyanates par cyclotrimérisation de fonctions isocyanates portées par des isocyanates ayant un squelette hydrocarboné ramifié, caractérisé en ce qu'il comprend les étapes suivantes :
a) fourniture d'isocyanates monomères de départ ;
b) élimination des gaz réactifs desdits monomères isocyanates;
c) éventuellement, fourniture d'un milieu réactionnel de départ comprenant lesdits isocyanates monomères de départ ;
d) addition d'un catalyseur de cyclotrimérisation consistant en un composé d'ammonium ou de phosphonium quaternaire, tel que défini ci-dessus ;
e) réaction jusqu'à obtention du taux de transformation désiré ; et
f) éventuellement élimination des monomères n'ayant pas réagi ; l'ordre des étapes b) et c) étant indifférent.

Ainsi, l'élimination des gaz réactifs peut avoir lieu sur les isocyanates de départ eux-mêmes ou après introduction de ceux-ci dans un milieu réactionnel éventuel comprenant un solvant approprié.

Il va de soi que lorsque la réaction est effectuée sans solvant, directement dans la masse des isocyanates, l'étape c) peut être omise.

Avantageusement, aucun substituant des composés "oniums" (ammonium ou phosphonium) n'est porteur de fonction hydroxyle (telle que phénol ou alcool).

En outre, selon une des mises en oeuvre préférées de la présente invention, aucun substituant ne présente moins de 2 atomes, avantageusement moins de 3 atomes de carbone.

Selon une autre mise en oeuvre préférée, l'ensemble des substituants comporte au moins 1 et au plus 2 radicaux de plus de 12 atomes, avantageusement de plus de 10 atomes, de préférence de plus de 6 atomes de carbone.

Les substituants sont avantageusement choisis parmi les alcoyles, y compris les aralcoyles et les cycloalcoyles. Il est préférable qu'ils ne portent pas de ramification en alpha et même en bêta. Il est également souhaitable qu'ils ne soient pas fonctionnalisés. Toutefois des fonctions éthers sont acceptables et notamment du type de celles qui sont issues de l'ouverture des époxydes par un alcool ou un alcoolate.

Selon la présente invention, sont considérés comme acides non seulement les acides protiques usuels mais encore l'eau et les alcools et tout composé pouvant subir l'arrachement d'un proton.

Les acides préférés sont essentiellement l'eau et les alcools, ce qui implique que les contre ions de l'ammonium quaternaire sont introduits sous la forme d'hydroxyde ou d'alcoolate.

Toutefois, on ne s'écartera pas de la présente invention en fabriquant *in situ* des composés de ce type. En effet, au cours de l'étude qui a mené à la présente invention, il a été démontré que la structure de l'ammonium ou du phosphonium jouait un rôle important dans le développement de la coloration subséquente à la réaction de polymérisation.

Il est apparu en particulier que la présence de méthyle et de composés de nature benzylique jouait un rôle néfaste sur la coloration de la composition après trimérisation.

Les résultats les meilleurs ont été obtenus lorsque les radicaux substituant l'ammonium ou le phosphonium identiques ou différents étaient choisis parmi propyle, butyle et pentyle, ces radicaux étant de préférence tous linéaires.

La présente invention vise la trimérisation de composés polyisocyanates en général et, de préférence, porteurs de deux fonctions isocyanates, désignés dans la présente description par isocyanates monomères.

Au cours de la réaction, d'autres composés peuvent toutefois être formés en fonction de la nature et de la formulation du catalyseur et, le cas échéant, du co-catalyseur tel que décrit dans la demande WO 99/23 128, notamment des dimères, aminooxadiazinediones, carbamates, allophanates, biurets, etc.

Quoique la présente invention puisse viser la trimérisation de monomères ramifiés avec des monomères non ramifiés, c'est-à-dire linéaires tels que l'HDI, elle vise principalement la trimérisation ou la trimérisation entre différents composés appartenant à la même famille des monomères ramifiés.

Par "monomères ramifiés" ainsi que cela a déjà été mentionné, on entend soit un monomère dont au moins une fonction isocyanate est en position cycloaliphatique, secondaire, tertiaire ou néopentylique. Ces monomères sont avantageusement des monomères cycloaliphatiques.

Ainsi, parmi ces produits, ceux qui sont préférés sont cycloaliphatiques. Ces monomères sont avantageusement tels qu'au moins l'une, avantageusement les deux fonctions isocyanates, soit distante du cycle le plus proche d'au plus un carbone et, de préférence, est reliée directement à lui. En outre, ces monomères cycloaliphatiques présentent avantageusement au moins une, de préférence deux fonctions isocyanates choisies parmi les fonctions isocyanates secondaire, tertiaire ou néopentylique.

Les meilleurs résultats sont obtenus quand la liberté conformationnelle du monomère cycloaliphatique est faible. Comme monomères susceptibles de donner de bons résultats, on peut citer à titre d'exemple les monomères suivants :
- les composés correspondant à l'hydrogénation du ou des noyaux aromatiques porteurs des fonctions isocyanates de monomères d'isocyanates aromatiques et notamment du TDI (totuènediisocyanate) et des diisocyanato-biphényles, le composé connu sous le sigle H₁₂MDI, les divers BIC [Bis(isocyanato-méthylcyclohexane)] et les cyctohexytdiisocyanates éventuellement substitués ;
   et surtout
- le norbornanediisocyanate, souvent désigné par son sigle NBDI ;
- l'isophoronediisocyanate, ou IPDI, ou plus précisément 3-isocyanatométhyl-3,5,5-triméthylcyclo-hexylisocyanate.

Les monomères de départ peuvent également être des produits d'oligomérisation d'isocyanates de plus faible masse moléculaire, ces produits d'oligomérisation étant porteurs de fonctions isocyanates. Dans ce cas, il n'est pas nécessaire de séparer l'oligomère non transformé du produit réactionnel formé.

Les travaux des inventeurs auteurs de la présente invention ont permis de découvrir que la polymérisation d'isocyanates du type décrit plus haut peut être effectuée avec un rendement élevé, et une coloration sensiblement réduite par la combinaison d'un catalyseur du type décrit ci-dessous et une étape d'élimination des gaz réactifs dissous dans le milieu réactionnel comprenant les monomères de départ sur lequel est réalisée la réaction de polymérisation ultérieure.

Par "gaz réactifs", on entend les gaz susceptibles d'interférer avec la réaction catalytique de polymérisation, notamment trimérisation, par opposition aux gaz inertes.

Il s'agit notamment de CO₂, mais également des gaz réactifs de l'air, c'est-à-dire les gaz autres que l'azote et les gaz rares, notamment l'oxygène, ainsi que du chlore ou éventuellement des composés volatils chlorés, provenant notamment de l'étape de phosgénation.

Les inventeurs ont par ailleurs déterminé que si la sensibilité de la réaction est influencée par la teneur en CO₂ dissous dans le milieu réactionnel, d'autres gaz présents notamment l'air ont une influence substantielle sur le déroulement de la réaction. Ainsi, l'élimination des gaz réactifs favorise grandement la réaction de trimérisation, même lorsque la teneur en CO₂ des monomères de départ est aussi faible que de l'ordre de 1 à 2 ppm.

Les inventeurs ont par ailleurs trouvé que la teneur en composés chlorés, définie par la teneur en chlore total, n'a qu'une influence minime sur la sensibilité de la réaction.

L'élimination des gaz réactifs peut être effectuée par tout moyen connu de l'homme du métier, notamment par mise sous vide, diffusion d'un gaz inerte ou encore mise en oeuvre de tamis moléculaires ou pièges chimiques, ou combinaison d'une ou plusieurs de ces méthodes.

Lorsque les monomères isocyanates de départ ont été stockés de manière prolongée généralement depuis plus de deux mois, conduisant à une argumentation des gaz réactifs dissous, il est généralement préférable d'éliminer les gaz réactifs dissous en soumettant le milieu réactionnel à un vide suivi de l'introduction d'un gaz inerte.

Le vide est généralement inférieur à 1.10⁴ Pa (100 mbar).

Lorsque les monomères de départ ont été stockés pendant une durée plus courte, l'introduction par diffusion dans le milieu réactionnel liquide d'un gaz inerte est généralement suffisante.

Le gaz inerte est avantageusement l'azote ou un gaz rare, par exemple l'argon. La diffusion est effectuée à une température de l'ordre de 0 à 70°C. Des températures plus élevées peuvent être utilisées.

La diffusion peut être réalisée par barbotage. Toutefois, le dégazage est d'autant plus efficace que la taille des bulles de gaz générées est petite. A cet effet, on adaptera les outils de diffusion de telle manière que l'élimination des gaz réactifs soit rapide sans entraîner de colmatage des dispositifs de diffusion mis en oeuvre.

La mise sous vide et/ou la diffusion sont effectuées pendant une durée suffisante pour abaisser le taux des gaz réactifs dans le milieu réactionnel de départ. En particulier, on considère que l'on à un bon dégazage quand le CO₂ dissous est inférieur à 20 ppm, de préférence inférieure à 2 ppm.

La mesure du taux de CO₂ ainsi que la teneur en gaz autres que le CO₂ sont avantageusement déterminés par des techniques analytiques classiques, notamment par chromatographie en phase gazeuse, en utilisant pour étalons des profils obtenus avec des injections de volumes variables de CO₂ pur et/ou des autres gaz purs.

Les conditions optimales de dégazage dépendent aussi des conditions de traitement des isocyanates après la synthèse de ceux-ci, en général par phosgénation, et des conditions de stockage.

En particulier l'opération de dégazage peut être réalisée après la phosgénation des amines, une fois que le mélange a été purifié et se soit stabilisé. Et ce dans les conditions ci-après. S'il est conservé dans de bonnes conditions (obscurité, température inférieure à 30°C dans des récipients hermétique à l'air et notamment à l'humidité), l'isocyanate monomère peut être utilisé dans le procédé de trimérisation même après une période de stockage prolongée (deux à quatre mois, voire plus).

Toutefois pour être sur d'obtenir une faible coloration, il est préférable de traiter les isocyanates stockés pendant une période prolongée comme décrit ci-après.

Des conditions avantageuses d'élimination des quantités de gaz réactifs dissous sont, dans le cas de quantités de monomères de départ de l'ordre de 500 g, entreposées depuis une période supérieure à deux mois, un vide de 1500 Pa (15 mbars) pendant une durée suffisante, généralement d'une heure à température ambiante, suivi par une diffusion de gaz inerte, par exemple d'azote par barbotage ou autre dans le liquide ou la masse pendant environ 30 minutes.

Pour des monomères de départ ayant été conservés pendant une période de temps moins longue, une simple diffusion d'azote généralement par barbotage pendant une durée de 30 minutes à température ambiante suffisante, pour la même quantité de monomères de départ.

Si le gaz à éliminer est fortement acide, c'est-à-dire ayant une valeur de pH inférieure à 3 (Handbook, 67^{ème} Ed., p D-146 of Chemistry and Physics), il est préférable d'effectuer le dégazage avant l'introduction du catalyseur, pour éviter l'empoisonnement de manière irréversible du catalyseur.

Inversement, lorsque le gaz à éliminer n'est pas fortement acide, en particulier dans le cas du CO₂, le dégazage peu être effectué avant ou après introduction du catalyseur et également pendant l'étape de trimérisation des isocyanates de départ.

Les inventeurs ont également montré que la formulation du catalyseur avait aussi une influence sur la cinétique de réaction. En particulier, dans le cas de formulations aqueuses, l'eau réagit avec l'isocyanate pour donner du biuret et du CO₂. Le CO₂ généré conduirait à une diminution de la vitesse de réaction. C'est pourquoi il est préférable de maintenir une diffusion continue de gaz inerte lorsqu'on utilise des formulations aqueuses de catalyseur.

Cela permet aussi de mettre en oeuvre des quantités très faibles de catalyseur et évite l'introduction successive de petites quantités de catalyseur pour maintenir la réactivité.

Les formulations aqueuses ou hydroalcooliques sont préférées, en raison de la solubilité des oniums dans ces milieux avant introduction dans le milieu réactionnel, due à l'hydrogène dissous dans ces solutions.

Les conditions opératoires du procédé de l'étape e) selon la présente invention peuvent être celles qui sont usuellement utilisées pour les trimérisations d'aliphatiques linéaires, en particulier la température peut être choisie entre 40 et 100°C, de préférence entre 50 et 90°C.

Un point de fonctionnement particulièrement satisfaisant se situe entre 60 et 80°C.

Il est par ailleurs possible de débuter le chauffage du milieu réactionnel avant la fin de l'étape b) d'élimination des gaz réactifs dissous, en particulier lorsque celle-ci consiste en un simple barbotage d'un gaz inerte.

Au cours de l'étude qui a mené à la présente invention, il a pu être montré que d'autres paramètres influaient sur la diminution de coloration. Les effets de ces paramètres sont favorables seuls ou en combinaison avec les autres paramètres dans la présente demande mentionnés ci-dessus.

Ainsi, il a été démontré qu'une distillation préalable des monomères de départ permettait de réduire la coloration développée au cours de la réaction de trimérisation.

Habituellement, on bloque les réactions de trimérisation utilisant des oniums quaternaires par chauffage. Au cours de l'étude qui a mené à la présente invention, il a été montré que cette technique de désactivation du catalyseur donnait de très mauvais résultats en ce qui concerne la coloration. Aussi, a-t-il été montré qu'il était préférable d'utiliser une autre technique, connue pour les aliphatiques linéaires, à savoir l'addition d'un acide de force élevée tel que les acides sulfoniques, par exemple les acides mésylique, tosylique, ou phosphonique, ou moyenne, de préférence un ester d'acide phosphoré.

Comme esters d'acides phosphorés, on peut citer les esters de phosphate, notamment les mono- et diesters. On peut également citer les phosphonates, de préférence monoestérifiés, et les phosphinates.

L'empoisonnement du catalyseur est toutefois de préférence réalisé au moyen d'esters acides de l'acide phosphorique, et notamment des esters phosphoriques de dialcoyle, en particulier tels que le phosphate de dibutyle et le phosphate de di-2-éthylhexyle.

La manière dont est introduit le catalyseur selon la présente invention joue également un rôle. Ainsi, lorsque le catalyseur est introduit dans un mélange aqueux, la coloration se développe moins que dans le cas où le catalyseur est introduit dans un mélange alcoolique.

Les solvants hydroalcooliques donnent toutefois d'assez bons résultats.

Pour éviter des zones d'emballement de la réaction, il est souhaitable d'introduire le catalyseur sous la forme d'une solution avec une bonne dilution massique. Des solutions de 0,5 % à 40 % en masse de catalyseurs donnent de bons résultats.

Les diluants doivent, d'une part, assurer la dissolution du composé ionique que constitue le catalyseur et, d'autre part, assurer la diffusion dans le milieu que constituent les monomères du mélange réactionnel. Des mélanges d'eau (0 à 50%, avantageusement de 5 à 40 %), de butanol (0 à 50%, de préférence 5 à 40 %) et d'alcool(s) lourd(s) (supérieur ou égal à C₈) ou d'alcools éthers (2-éthoxy éthylèneglycol) (qsp 100%) donnent de bons résultats.

Bien qu'il soit préférable de travailler en utilisant des solutions relativement diluées de catalyseurs, il est possible, selon la présente invention, d'utiliser des solutions très concentrées de catalyseurs, à condition de réaliser l'introduction à basse température, avantageusement à température ambiante ou de préférence inférieure à la température ambiante, et d'homogénéiser avant d'enclencher le processus de chauffe du mélange réactionnel.

En général, la trimérisation est menée jusqu'au taux de transformation désiré. Pour des raisons économiques évidentes, on préfère arrêter la réaction à des taux de transformation des fonctions isocyanates d'au moins 5 % et de façon préférée entre 10 et 50 %. A des taux élevés supérieurs à 50 %, les polyisocyanates présentent généralement une viscosité élevée difficilement compatible avec les conditions d'application ultérieures. Une fois la réaction de polymérisation arrêtée, on élimine en général le reste des monomères par évaporation ou distillation sur film mince et sous vide poussé.

La quantité de catalyseur utilisée est avantageusement comprise entre 10⁻⁵ et 10⁻² fois la masse totale des monomères isocyanates introduits, de préférence 10⁻⁴ à 10⁻³, exprimé en quantités massiques du catalyseur sans solvant. Dans le cas des oligomères, on préfère des quantités de catalyseurs telles que le rapport masse du catalyseur/masse des fonctions isocyanates soit compris entre 10⁻⁵ et 10⁻².

La présente invention vise aussi des compositions comportant :
- de 20% à 80% en masse de composé(s) à cycles isocyanurates réalisés à partir de monomère(s) cycloaliphatique(s) ;
- de 10⁻⁶ à 10⁻² d'un ammonium ou d'un phosphonium quaternaire dont la somme des carbones est au plus égale à 25 et au moins égale à 15, et dont aucun substituant ne présente moins de 2 atomes, avantageusement de 3 atomes de carbone ;
- de 20% à 80% de monomère(s) cycloaliphatique(s),
et présentant une coloration ramenée au pourcentage en masse de composé(s) à cycles isocyanurates d'au plus 1 Hazen, avantageusement d'au plus 0,75 hazen, de préférence d'au plus 0,50 Hazen.

Les exemples non limitatifs suivants illustrent l'invention.

La méthode de dosage des fonctions NCO est réalisée selon la norme AFNOR NFT 52-132 (réaction des fonctions isocyanates avec la dibutylamine en excès et dosage de la dibutylamine en retour par l'acide chlorhydrique).

### Méthode de mesure du CO₂

Le CO₂ est mesuré selon une technique de chromatographie en phase gazeuse.

### Conditions d'analyse CPG

| | |
|---|---|
| chromatographe | : Hewlett-Packard HP5890 n° 6658 05 84 |
| colonne inox | : PORAPAK T 80-100 Mesh L:3m d.i.:2 mm |
| température four | : 50°C isotherme |
| gaz vecteur | : hélium |
| débit colonne | : 46,7 ml/mn à 50°C pour P-243,7 Kpa (Fpmeter) (colonne + référence:66.7) |
| injection | : dans tube en U de stripping verre |
| température détecteur | : 230°C |
| détecteur | : catharomètre basse sensibilité range 0 |
| volume injecté | : 0,5 ml d'échantillon liquide injecté dans le tube verre de stripping |
| étalonnage | : étalonnage externe par des injections de volumes variables de CO₂ pur. Les volumes faibles (inférieurs à 0,1 ml) sont obtenus par une dilution de 1 ml de CO₂/227 ml d'hélium. |

La méthode de dosage du chlore hydrolysable est effectuée selon la norme AFNOR NF T 52-135.

### Exemple 1 :

800 g d'IPDI sont chargés dans un réacteur de 1 l et agités sous courant d'argon. Le catalyseur est introduit dans les proportions indiquées ci-après à température ambiante. L'agitation est maintenue et le milieu réactionnel est mené à la température de réaction en général à 68°C. L'évolution de la réaction est suivie par titration des fonctions résiduelles NCO. La choline, le TMBA, à savoir le triméthylbenrylammonium, le TBA, à savoir le tétrabutylammonium, le TBP (tétrabutylphosphonium), le TOMA (méthyltrioctylammonium), le phényl-triméthylammonium (TMPA) et le TOEP (éthyltrioctylphosphonium) ont été testés sous la forme d'hydroxydes dans les conditions opératoires ci-après : température 68°C. Les autres conditions opératoires, les quantités de catalyseurs et les résultats sont rassemblés dans le tableau suivant.

| Catalyseur | Solvant (% poids cata) | [catalyseur] % poids | TT(IPDI) % | Coloration MR brute (Hazen) |
|---|---|---|---|---|
| Choline, OH (C)* | Eau (50%) | 0,05 | 30 | 159 |
| TMBA, OH (C)* | eau/MeOH (18%) | 0,12 | 34 | 84 |
| TBA, OH | Eau (26%) | 0,35 | 48 | 52 |
| TBA, OH | MaOH (26%) | 0,03 | 29 | 47 |
| TBP, OH | Eau (50%) | 0,35 | 21 | 35 |
| TMPA, OH (C)* | Eau (24%) | 0,35 | 3 | < 5 |
| TOMA OH (C)* | Eau (50%) | 0,35 | 24 | 228 |
| TMHA, OH | MeOH (50%) | 0,35 | 20 | 35 |
| TOEP, OH(C)* | MaOH (26%) | 0,35 | 9 | 60 |

| | | | | |
|---|---|---|---|---|
| *(C) = comparatif | | | | |

Ce tableau montre que la choline donne de bons rendements mais une coloration médiocre, que le TMBA donne également une coloration médiocre, le TMPA donne un rendement extrêmement faible, l'aliquat donne un rendement correct mais une très forte coloration.

### Exemple 2 : Rôle de l'empoisonnement du catalyseur

Des essais, dans les conditions précédentes avec de l'IPDI redistillé, ont été réalisés avec comme catalyseur du tétrabutylammonium sous forme d'hydroxyde dissous dans l'eau à raison de 26%. La quantité de catalyseur (sec) utilisé est de 0,2% et 0,4% dans le cas du catalyseur choline bic, et la température de réaction est de 68°C. Les essais sont rassemblés dans le tableau suivant.

| Essai | TT (IPDI)% | Coloration MR (Hazen) |
|---|---|---|
| Blocage 120°C (C)* | - | Jaune (AB)*** |
| Blocage DBP***** | 44 | 9 (AB)*** |
| Blocage A.P.T.S.** | 50 | 6 (AB)*** |
| | 49 | < 5 (PB)**** |
| Blocage DBP | 51 | 8 (AB)*** |
| | 50 | < 5 (PB)**** |
| cata choline, bic/eau | 25 | 46 (AB)*** |
| Blocage DBP | 25 | 48 (PB)**** |

| | | |
|---|---|---|
| * (C) = comparatif | | |
| ** A.P.T.S. = acide paratoluènesulfonique | | |
| *** (AB) = avant blocage | | |
| **** (PB) = après blocage | | |
| ***** (DBP) = phosphate de dibutyle | | |

Lors des différents essais réalisés, la procédure consiste, lorsque le TT(IPDI) souhaité est atteint, à ajouter la DBP directement dans le milieu réactionnel, sous agitation, à la température de réaction. Après 30 minutes de traitement, on réalise un nouvel échantillonnage avec titrage des fonctions NCO libres et mesure de la coloration.

La quantité de DBP correspond à 1 équivalent molaire, par rapport à la quantité initiale de catalyseur introduite. Dans le cas de la catalyse TBA, OH/eau, on introduit alors 0,17% de DBP, en poids par rapport à l'IPDI initial. Dans le cas de la catalyse choline, bic/eau, on introduit 0,05% en poids.

Dans tous les cas, avec les catalyseurs choline et TBA, le blocage par le DBP apparaît efficace, avec un arrêt de la réaction confirmé après 30 minutes de contact.

### Exemple 3 :

### Réactifs (% exprimés en poids/poids) :

- IPDI = 504 g, la teneur initiale en CO₂ est évaluée à 45 mg/kg d'IPDI, l'IPDI est distillé avant usage
- catalyseur hydroxyde de tétrabutylammonium (TBA OH), solution catalytique 6% TBA OH / 2-éthylhexanol-butanol-eau (selon un rapport massique 83 : 12 : 5) = 1,47 g
- solution de blocage, 8% APTS (acide p-toluène sulfonique) /diéthyl éther d'éthylèneglycol = 1,06 g

### Mode opératoire

La réaction est effectuée dans un réacteur de 1 l avec agitation mécanique, régulation de la température et bullage d'argon par canne plongeante.

Après introduction dans le réacteur, l'IPDI est chauffé 1 h à 68°C, sous agitation, vide partiel de 3900 Pa (39 mbar) et bullage d'argon. La teneur de CO₂ est alors évaluée par la méthode décrite ci-dessus inférieure à 2 mg/kg d'IPDI

La solution catalytique est introduite en 26 minutes. Le milieu réactionnel est maintenu à une température de 68°C, sous agitation. L'avancement de la réaction est suivi par titration des fonctions isocyanates résiduelles. Après une durée totale de 1 h 15 min, le taux de transformation de l'IPDI atteint 41%, la solution de blocage est introduite et, 15 minutes après, la masse réactionnelle est refroidie à température ambiante. La coloration de la masse réactionnelle est évaluée à 12 Hazen.

La masse réactionnelle est distillée sur évaporateur à couche mince, à une température de 191 °C et un vide inférieur à 30 Pa (0,3 mbar).

Le produit final formulé à 70 % d'extrait sec dans l'acétate de n-butyle présente une apparence limpide et une coloration de 40 Hazen, un taux de NCO de 12,5% et une viscosité de 342 mPa.s.

### Exemple 4 :

### Réactifs (% exprimés en poids/poids) :

- IPDI = 504 g, la teneur initiale en CO₂ est évaluée inférieure à 2 mg/kg d'IPDI, l'IPDI est distillé avant usage
- catalyseur hydroxyde de tétrabutylammonium (TBA OH), solution catalytique 6% TBA OH / 2-éthylhexanol-butanol-eau (selon un rapport massique 83 : 12: 5) = 1,71 g
- solution de blocage, 10% APTS / 2-éthylhexanol = 0,76 g

### Mode opératoire

La réaction est effectuée dans un réacteur de 1 l avec agitation mécanique, régulation de la température et bullage d'argon par canne plongeante.

Après introduction dans le réacteur, l'IPDI est maintenu 1 h à 25°C, sous agitation et bullage d'argon.

La solution catalytique est introduite en 20 minutes. Le milieu réactionnel est chauffé à une température de 50°C, sous agitation. L'avancement de la réaction est suivi par titration des fonctions isocyanates résiduelles. Après une durée totale de 3 h 7 min, le taux de transformation de l'IPDI atteint 47%, la solution de blocage est introduite et, 15 minutes après, la masse réactionnelle est refroidie à température ambiante. La coloration de la masse réactionnelle est évaluée à 8 Hazen.

La masse réactionnelle est distillée sur évaporateur à couche mince, à une température de 191°C et un vide inférieur à 30 Pa (0,3 mbar).

Le produit final formulé à 70 % d'extrait sec dans l'acétate de n-butyle présente une apparence limpide et une coloration de 13 Hazen, un taux de NCO de 12,5% et une viscosité de 885 mPa.s.

### Exemple 5 (comparatif) :

### Réactifs (% exprimés en poids/poids) :

- IPDI= 35 g
- catalyseur hydroxyde de 2-hydroxyéthyltriméthylammonium, en solution à 45% dans le méthanol = 0,032 g

### Mode opératoire

La réaction est effectuée dans un réacteur de 0,1 l avec agitation mécanique, régulation de la température et bullage CO₂ par canne plongeante dans les mêmes conditions que l'exemple 3.

La réaction est réalisée à 80°C, après une durée de 1 h 30 min, le taux de transformation de l'IPDI atteint 35%. La masse réactionnelle est refroidie à température ambiante et la coloration est évaluée à 191 Hazen.

## Revendications

1. Procédé de cyclotrimérisation de monomères isocyanates aliphatiques par action d'un catalyseur à base d'ammonium ou de phosphonium quaternaire **caractérisé par le fait que** lesdits monomères sont ramifiés et **par le fait que** lesdits oniums sont choisis parmi ceux dont la somme des carbones est au plus égale à 30 et au moins égale à 12 ; dont le contre-ion est choisi parmi les anions correspondant à des acides faibles dont le p*K*_{A} est au moins égal à 8, de préférence à 10, plus préférentiellement à 12; dont les substituants sont aliphatiques et ne possèdent pas d'insaturation en bêta ; et que lesdits oniums sont introduits sous forme de mélange aqueux comportent au plus 2 radicaux de plus de 6 atomes, avantageusement de plus de 10 atomes, de préférence de plus de 12 atomes de carbone, et qu'aucun substituant dudit onium n'est porteur de fonction hydroxyle.

2. Procédé selon la revendication 1 par (cyclo)trimérisation de fonctions isocyanates portées par des monomères isocyanates aliphatiques ayant un squelette hydrocarboné ramifié, **caractérisé en ce qu'**il comprend les étapes suivantes :
a) fourniture desdits monomères;
b) élimination des gaz réactifs desdits monomères;
c) éventuellement, fourniture d'un milieu réactionnel de départ comprenant lesdits monomères ;
d) addition d'un catalyseur de (cyclo)trimérisation tel que défini à la revendication 1 ;
e) réaction jusqu'à obtention du taux de transformation désiré ; et
f) éventuellement élimination des monomères n'ayant pas réagi ; l'ordre des étapes b) et c) étant indifférent.

3. Procédé selon la revendication 1 ou 2, **caractérisé en ce que** le mélange aqueux est un mélange hydroalcoolique, avantageusement un mélange comprenant au moins 5% d'eau et au plus 50% d'eau, de préférence au plus 40% d'eau.

4. Procédé selon l'une des revendications 1 à 3, **caractérisé par le fait que** ledit contre-ion est un hydroxyde ou un alcoolate ou l'un des anions qu'ils induisent dans le milieu réactionnel.

5. Procédé selon l'une des revendications 1 à 4, **caractérisé par le fait que** la teneur en catalyseur du mélange réactionnel est comprise entre 10⁻⁵ et 10⁻² fois la masse totale des monomères isocyanates.

6. Procédé selon l'une des revendications 1 à 4, **caractérisé par le fait qu'**aucun substituant dudit onium ne présente moins de 2 atomes, avantageusement moins de 3 atomes de carbone.

7. Procédé selon l'une des revendications 1 à 6, **caractérisé par le fait que** l'ensemble des substituants dudit onium comporte au moins un et au plus deux radicaux de plus de 12 atomes, avantageusement de 10, de préférence de 6 atomes de carbone.

8. Procédé selon l'une des revendications 1 à 7, **caractérisé en ce que** la réaction est arrêtée par l'addition d'un acide moyen ou fort choisi parmi les acides sulfoniques, avantageusement liposolubtes ; et les acides phosphoriques, avantageusement un mono ou un diester d'acide phosphorique.

9. Procédé selon l'une des revendications 1 à 8, **caractérisé par le fait que** les fonctions isocyanates sont portées par des isocyanates monomères cycloaliphatiques.

10. Procédé selon la revendication 9, **caractérisé par le fait que** les fonctions isocyanates sont portées par l'isophorone diisocyanate.

11. Procédé selon l'une des revendications 2 à 10, **caractérisé en ce que** l'élimination des gaz réactifs du milieu réactionnel est effectuée par diffusion d'un gaz inerte.

12. Procédé selon l'une des revendications 2 à 10, **caractérisé en ce que** l'élimination des gaz réactifs est réalisée par mise sous vide du milieu réactionnel à un vide inférieur à 1.10⁴ Pa, suivi par diffusion d'un gaz inerte.

13. Procédé selon l'une quelconque des revendications 2 à 10, **caractérisé en ce que** dans l'étape b), on élimine le CO₂.

14. Procédé selon l'une quelconque des revendications 2 à 10, **caractérisé en ce que** dans l'étape b), on élimine l'air dissous dans le liquide ou la masse des monomères de départ.

15. Compositions de polyisocyanates-isocyanurates obtenus par le procédé selon l'une des revendications 1 à 14, présentant une faible coloration, dont la valeur est au plus de 52 Hazen.

## Patentansprüche

1. Verfahren zur Cyclotrimerisierung von aliphatischen Isocyanat-Monomeren durch Einwirkung eines Katalysators auf der Basis von quaternärem Ammonium oder Phosphonium, **dadurch** charakterisiert, dass die Monomere verzweigt sind und dass die Oniums ausgewählt werden aus solchen, deren Summe von Kohlenstoffatomen höchstens gleich 30 ist und mindestens gleich 12 ist; deren Gegenion ausgewählt wird aus Anionen, die schwachen Säuren entsprechen, deren pKA mindestens gleich 8, bevorzugt gleich 10, noch bevorzugter gleich 12 ist; deren Substituenten aliphatisch sind und keine Ungesättigtheit in β-Position aufweisen; und dass die Oniums in Form einer wässrigen Lösung eingeführt werden, höchstens 2 Reste mit mehr als 6 Kohlenstoffatomen, vorteilhafterweise mehr als 10 Kohlenstoffatomen, bevorzugt mehr als 12 Kohlenstoffatomen umfassen, und dass kein Substituent des Onium eine Hydroxylfunktion aufweist.

2. Verfahren nach Anspruch 1 durch (Cyclo)trimerisierung von Isocyanatfunktionen, die sich an aliphatischen Isocyanat-Monomeren befinden, welche ein verzweigtes Kohlenwasserstoffgerüst aufweisen, **dadurch** charakterisiert, dass es die folgenden Schritte umfasst:
a) Bereitstellung dieser Monomere;
b) Entfernung von reaktiven Gasen aus diesen Monomeren;
c) gegebenenfalls Bereitstellung eines Ausgangs-Reaktionsmediums, welches diese Monomere umfasst;
d) Zugabe eines (Cyclo)trimerisierungs-Katolysators wie in Anspruch 1 definiert;
e) Reaktion bis zum Erhalt des gewünschten Umsetzungsgrades; und
f) gegebenenfalls Entfernung von unreagierten Monomeren;
wobei die Reihenfolge der Schritte b) und c) gleichgültig ist.

3. Verfahren nach Anspruch 1 oder 2, **dadurch** charakterisiert, dass aie wässrige Mischung eine wässrige alkoholische Mischung ist, vorteilhafterweise eine Mischung, die mindestens 5 % Wasser und höchstens 50 % Wasser umfasst, bevorzugt höchstens 40 % Wasser.

4. Verfahren nach einem der Ansprüche 1 bis 3, **dadurch** charakterisiert, dass das Gegenion ein Hydroxid oder ein Alkoholat oder eines der Anionen ist, die sie im Reaktionsmedium erzeugen.

5. Verfahren nach einem der Ansprüche 1 bis 4, **dadurch** charakterisiert, dass der Katalysator-Gehalt der Reaktionsmischung das 10⁻⁵ bis 10⁻² fache der Gesamtmasse der Isocyanat-Monomeren beträgt.

6. Verfahren nach einem der Ansprüche 1 bis 4, **dadurch** charakterisiert, dass kein Substituent des Onlum weniger als 2 Kohlenstoffatome, vorteilhafterweise weniger als 3 Kohlenstoffatome aufweist.

7. Verfahren nach einem der Ansprüche 1 bis 6, **dadurch** charakterisiert, dass die gesamten Substituenten des Onium mindestens einen und höchstens zwei Reste mit mehr als 12 Kohlenstoffatomen, vorteilhafterweise mehr als 10, bevorzugt mehr als 6 Kohlenstoffotomen aufweisen.

8. Verfahren nach einem der Ansprüche 1 bis 7, **dadurch** charakterisiert, dass die Reaktion durch Zugabe einer mittleren oder starken Säure beendet wird, die ausgewählt wird aus Sulfonsäuren, vorteilhafterweise tettlöslichen, und Phosphorsäuren, vorteilhafterweise einem Phosphorsäuremono- oder -diester.

9. Verfahren nach einem der Ansprüche 1 bis 8, **dadurch** charakterisiert, dass die isocyanatfunktionen sich an cycloaliphatischen monomeren Isocyanaten befinden.

10. Verfahren nach Anspruch 9, **dadurch** charakterlsiert, dass die Isocyanatfunktionen sich an Isophorondiisocyanat befinden.

11. Verfahren nach einem der Ansprüche 2 bis 10, **dadurch** charakterisiert, dass die Entfernung der reaktiven Gase aus dem Reaktionsmedium durch Diffusion eines Inertgases bewirkt wird.

12. Verfahren nach einem der Ansprüche 2 bis 10, **dadurch** charakterisiert, dass die Entfernung der reaktiven Gase durch Anlegen eines Vakuums an das Reaktionsmedium von weniger als 1 x 10⁴ Pa, gefolgt von Diffusion eines Inertgases bewirkt wird.

13. Verfahren noch einem der Ansprüche 2 bis 10, **dadurch** charakterisiert, dass man in Schritt b) CO₂ entfernt.

14. Verfahren nach einem der Ansprüche 2 bis 10, **dadurch** charakterisiert, dass man in Schritt b) die Luft entfernt, die in der Flüssigkelt oder der Masse der Ausgangsmonomere gelöst ist.

15. Polyisocyanat-Isocyanurat-Zusammensetzungen, erhalten durch das Verfahren noch einem der Ansprüche 1 bis 14, welche eine schwache Färbung aufweisen, deren Wert höchstens 52 Hazen beträgt.

## Claims

1. Method for cyclotrimerisation of aliphatic isocyanate monomers by the action of a catalyst based on quaternary ammonium or phosphonium, **characterised in that** said monomers are branched and **in that** said oniums are selected from those of which the sum of the carbons is at most equal to 30 and at least equal to 12; the counterion of which is selected from the anions corresponding to weak acids of which the pK_{A} is at least equal to 8, preferably to 10 and more preferably to 12; the substituents of which are aliphatic compounds and do not have an unsaturation in the beta position; and **in that** said oniums are introduced in the form of an aqueous mixture and comprise at least 2 radicals of more than 6, advantageously more than 10, preferably more than 12 carbon atoms, and **in that** no substituent of said onium carries a hydroxyl function.

2. Method according to claim 1 for (cyclo) trimerisation of isocyanate functions carried by aliphatic isocyanate monomers having a branched hydrocarbon backbone, **characterised in that** it comprises the following steps:
a) providing said monomers;
b) removing the reactive gases from said monomers;
c) possibly, providing a starting reaction medium comprising said monomers;
d) adding a (cyclo) trimerisation catalyst such as defined in claim 1;
e) reaction until the desired degree of conversion is obtained; and
f) possibly removing monomers which have not reacted;
the order of steps b) and c) being immaterial.

3. Method according to claim 1 or 2, **characterised in that** the aqueous mixture is a hydroalcoholic mixture, advantageously a mixture comprising at least 5% water and at most 50% water, preferably at most 40% water.

4. Method according to one of claims 1 to 3, **characterised in that** said counterion is a hydroxide or an alcoholate or one of the anions which they induce in the reaction medium.

5. Method according to one of claims 1 to 4, **characterised in that** the catalyst content of the reaction mixture is between 10⁻⁵ and 10⁻² times the total mass of the isocyanate monomers.

6. Method according to one of claims 1 to 4, **characterised in that** no substituent of said onium has less than 2, advantageously less than 3 carbon atoms.

7. Method according to one of claims 1 to 6, **characterised in that** all of the substituents of said onium include at least one and at most two radicals of more than 12, advantageously more than 10, preferably more than 6 carbon atoms.

8. Method according to one of claims 1 to 7, **characterised in that** the reaction is stopped by the addition of a medium or strong acid selected from sulphonic acids, advantageously liposoluble; and phosphoric acids, advantageously a monoester or diester of phosphoric acid.

9. Method according to one of claims 1 to 8, **characterised in that** the isocyanate functions are carried by cycloaliphatic isocyanate monomers.

10. Method according to claim 9, **characterised in that** the isocyanate functions are carried by isophorone diisocyanate.

11. Method according to one of claims 2 to 10, **characterised in that** the reactive gases are removed from the reaction medium by diffusion of an inert gas.

12. Method according to one of claims 2 to 10, **characterised in that** the reactive gases are removed by subjecting the reaction medium to a vacuum lower than 1.10⁴ Pa, followed by diffusion of an inert gas.

13. Method according to any one of claims 2 to 10, **characterised in that** the CO₂ is removed in step b).

14. Method according to any one of claims 2 to 10, **characterised in that** the air dissolved in the liquid or the mass of the starting monomers is removed in step b).

15. Compositions of polyisocyanates-isocyanurates obtained by the method according to one of claims 1 to 14, which have a slight colouring, the value of which is at most 52 Hazen.
